# EUROPÄISCHE PATENTSCHRIFT

(11) **EP 2 408 349 B1**
(45) Veröffentlichungstag und Bekanntmachung des Hinweises auf die Patenterteilung: **30.08.2017**
(21) Anmeldenummer: 10722569.0
(22) Anmeldetag: 15.03.2010
(51) Int. Cl.: A61B 5/00

(54) **ÜBERWACHUNGSVORRICHTUNG UND ÜBERWACHUNGSVERFAHREN FÜR BLUTZUCKERWERTE**
MONITORING DEVICE AND MONITORING METHOD FOR BLOOD GLUCOSE VALUES
DISPOSITIF DE SURVEILLANCE ET PROCÉDÉ DE SURVEILLANCE DES TAUX DE GLUCOSE DANS LE SANG

(30) Priorität: 16.03.2009 DE 102009003625
(43) Veröffentlichungstag der Anmeldung: 25.01.2012
(73) Patentinhaber: Roche Diabetes Care GmbH, 68305 Mannheim (DE); F. Hoffmann-La Roche AG, 4070 Basel (CH)
(72) Erfinder: FAHIMI, Sohrab M., 10961 Berlin (DE)
(74) Vertreter: Bakhtyari, Arash
(86) Internationale Anmeldenummer: PCT/DE2010/075027
(87) Internationale Veröffentlichungsnummer: WO 2010/105619

(56) Entgegenhaltungen:
- WO-A2-2008/009737
- US-A1- 2003 076 983
- US-A1- 2003 208 113

## Beschreibung

Die Erfindung betrifft eine Überwachungsvorrichtung und ein Überwachungsverfahren für Blutzuckerwerte.

Diabetespatienten müssen ihren eigenen Blutzuckerspiegel in regelmäßigen Abständen messen. In der Regel ist auch eine Messung kurz vor der Einnahme einer Mahlzeit notwendig. Für die Überwachung einer Diabetes-Diät und insbesondere bei Anwendung einer Insulintherapie ist es zudem notwendig, für jede Mahlzeit die in ihr enthaltene Menge an relevanten Kohlehydraten abzuschätzen. Die Kohlehydratmenge wird in Broteinheiten beziehungsweise Kohlenhydrateinheiten gemessen. In der Regel wird der Patient beauftragt, ein Ernährungsprotokoll zu führen, indem er die von ihm eingenommenen Mahlzeiten beschreibt oder zumindest die hierfür geschätzten Broteinheiten notiert.

Moderne Blutzuckermessgeräte, auch Glukosemeter genannt, sind in der Lage, eine Reihe von Blutzuckermesswerten so zu speichern, dass sie anschließend ausgelesen werden können. Ein behandelnder Arzt oder Diabetestherapeut kann dann die ausgelesenen Werte im zeitlichen Verlauf betrachten und dem Patienten Anweisungen für den weiteren Verlauf der Diabetes-Diät beziehungsweise der Insulintherapie geben. Hierzu muss auch das vom Patienten geführte Ernährungsprotokoll berücksichtigt werden, dem hierbei somit eine wesentliche Rolle zukommt.

Nachteilig wirkt sich bei der Erstellung des Ernährungsprotokolls die Tatsache aus, dass der Patient die Protokollunterlagen jederzeit bei sich tragen muss. Alternativ muss er die zu sich genommene Nahrungsmenge und -art notieren und anschließend in das Protokoll übertragen. Dies erscheint dem Patienten oft als mühsam und umständlich, was einerseits zu einer Beeinträchtigung seiner Lebensqualität führt, und anderseits auch regelmäßig die Sorgfalt beim Erstellen des Ernährungsprotokolls beeinträchtigt. Das kann dazu führen, dass einzelne Nahrungsmittel oder ganze Speisen nicht aufgeführt werden, oder falsch oder unzureichend beschrieben werden.

US 2003/ 208113 A1 offenbart ein System, welches einen Diabetespatienten dabei unterstützen soll, sein Blut zuckerwert möglichst konstant zu halten. Das System umfasst hierzu ein tragbares Kommunikationsgerät, welches als PDA bezeichnet wird. Das PDA ist über eine Funkverbindung mit unterschiedlichen Sensoren verbunden, darunter mit einem Blutzuckermessgerät. Ferner umfasst das PDA in einer herausgestellten Ausführungsform eine Digitalkamera, welche dazu dient, Bilder von Speisen aufzunehmen, die der Patient vorhat, zu sich zu nehmen. Mittels Auswertung der Bilder können dann die vom Patienten aufgenommenen Nährstoffe ermittelt und gespeichert werden. Ziel ist es, ein Ernährungsprotokoll ("diet log") für den Patienten zu erstellen, auf welches das System zugreifen kann.

In WO 2008/009737 A2 wird ein System zur Blutzuckerüberwachung und zur Bestimmung von blutzuckerbeeinflussenden Faktoren für einen Benutzer offenbart. Das System umfasst einen Rechner, an dem unterschiedliche Geräte angeschlossen werden können, insbesondere ein tragbares Handgerät und ein Blutzuckermessgerät. In einer besonderen Ausführungsform, kann das tragbare Handgerät eine Digital kamera enthalten, mittelswelcher der Benutzer Fotos von Speisen aufnehmen kann, die er verspeist. Aufgrund dieser Fotos können Parameter ermittelt werden, welche den Blutzuckerwert des Benutzers beeinflussen.

In US 2003/076983 wird ein Gerät beschrieben, welches in der Lage ist, ein Foto von einer auf einem Teller angeordneten Mahlzeit aufzunehmen und anhand des Fotos den Nährstoffinhalt der Mahlzeit zu ermitteln. Das Gerät weist hierzu eine Digital kamera und eine Bildauswertungseinheit auf.

Es ist Aufgabe der Erfindung, eine Vorrichtung und ein Verfahren bereitzustellen, um die Erstellung eines Ernährungsprotokollsfür einen Diabetespatienten zu unterstützen oder zu erleichtern.

Die Aufgabe wird gemäß der Erfindung durch eine Überwachungsvorrichtung mit den Merkmalen des Anspruchs 1 und durch ein Überwachungsverfahren mit den Merkmalen des Anspruchs 11 gelöst. Vorteilhafte Weiterbildungen der Erfindung sind in den Unteransprüchen aufgeführt.

Die Erfindung beruht auf den Gedanken, einem Nutzer, insbesondere einem Diabetespatienten die Möglichkeit zu geben, die von ihm eingenommenen Lebensmittel und Speisen vor dem Verzehr zuvor zu fotografieren oder auf einer anderen Weise ein Bild davon zu machen. Derartige Bilder von den verzehrten Speisen können das Ernährungsprotokoll ergänzen oder sogar vollständig ersetzen. Da zudem die Blutzuckermesseinheit und die Bildaufnahmeeinheit in einer Überwachungsvorrichtung kombiniert sind, können sie auf die speziellen Bedürfnisse von Diabetespatienten abgestimmt sein. Beispielsweise kann die Bildaufnahmeeinheit speziell für die Nahaufnahmen konzipiert oder optimiert sein.

Bei der Blutzuckermesseinheit handelt es sich um eine Vorrichtung zur Bestimmung des Glucosegehaltes im Blut mittels Untersuchung einer Blutprobe. Eine derartige Messeinheit kann beispielsweise auf kalorimetrischer, fotometrischer, amperometrischer und / oder elektrochemischer Basis messen. Es sich jedoch auch nicht-invasive Messmet hoden geeignet. Als Bildaufnahmeeinheiten kommen insbesondere digitale Fotokameras infrage. Es können jedoch auch andere Formen der Bildaufnahme eingesetzt werden, wie beispielsweise mittels Filmkameras und dergleichen.

Es ist eine Zuordnungseinheit vorgesehen, welche ausgebildet ist zum Zuordnen der Bilder zu zugehörigen Blutzuckermesswerten. Die Zuordnung kann beispielsweise anhand einer zeitlichen Reihenfolge geschehen, in der die Bilder erfasst und die Blutzuckermesswerte gemessen wurden. Beispielsweise können eine oder mehrere aufgenommene Bilder einem zeitlich später gemessenen Blutzuckermesswert zugeordnet werden. Die Zuordnung kann mittels einer Zuordnungstabelle erfolgen. Alternativ können Zuordnungsparameter mit jedem Bild und / oder mit jedem Messwert verknüpft werden, beispielsweise eine laufende Nummer. Schließlich ist es möglich, dass ein Nutzer der Überwachungsvorrichtung mittels einer Eingabeeinheit die Zuordnung zwischen Blutzuckermesswerten und Bildern bewirkt oder steuert. Bei der Eingabeeinheit kann es sich beispielsweise um ein Touchscreen handeln, auf dem auch die gemessenen Blutzuckermesswerte und / oder die erfassten Bildern angezeigt werden können.

In einer vorteilhaften Weiterbildung ist vorgesehen, dass die Zuordnungseinheit ausgebildet ist zum Zuteilen von Zeitstempeln zu den Bildern und den Blutzuckermesswerten. Die Zuordnung kann somit anhand der zeitlichen Abfolge erfolgen, in der die Bilder erfasst und die Blutzuckermesswerte gemessen wurden. Zusätzlich zu der oben beschriebenen Reihenfolge, kann hier auch eine Aussage darüber getroffen werden, welcher zeitliche Abstand zwischen der Messung eines oder mehrerer Blutzuckermesswerte und dem Verzehr eines Nahrungsmittels liegt, solange sichergestellt ist, dass zwischen Bilderfassung und Verzehr kein wesentlicher Zeitunterschied liegt.

Gemäß einer zweckmäßigen Ausgestaltung ist eine Bildanzeigeeinheit vorgesehen, welche ausgebildet ist zum Anzeigen der Bilder und / oder der Blutzuckermesswerte. Hierbei kann es sich beispielsweise um eine bei Digitalkameras übliche, digitale Anzeige handeln. Neben den erfassten Bildern und den gemessenen Blutzuckermesswerten, können mittels der Bildanzeigeeinheit auch weitere relevante Parameter angezeigt werden, wie beispielsweise Zeitabstand zu einer vorhergehenden Blutzuckermesswertmessung oder ein Speicher- oder Batterieladezustand.

Bevorzugterweise ist vorgesehen, dass die Blutzuckermesseinheit, die Bildaufnahmeeinheit, die Zuordnungseinheit und / oder die Bildanzeigeeinheit in einem gemeinsamen Gehäuse untergebracht sind. Eine derartige Vereinigung dieser Einheiten in einem gemeinsamen Gehäuse hat den Vorteil, dass die Überwachungsvorrichtung einfach handhabbar ist. Zudem können die einzelnen Einheiten bei der Herstellung optimal aufeinander abgestimmt sein.

Bei einer zweckmäßigen Ausführungsform ist vorgesehen, dass das Gehäuse als mobiles Handgerät ausgebildet ist. Ein derartiges Handgerät, auch Handheld genannt, kann ein Diabetespatient zu jeder Zeit bei sich tragen. Er braucht sich lediglich daran zu erinnern, dieses eine Handgerät mit sich zu führen; zusätzliche Unterlagen für ein schriftliches Ernährungsprotokoll sind prinzipiell nicht notwendig. Das Handgerät kann zudem weitere Funktionen in sich vereinen. Insbesondere kann es ein Kommunikationsmodul oder sogar sämtliche Funktionen eines Mobiltelefons aufweisen, um etwa erfasste Bilder und / oder gemessene Blutzuckermesswerte zu übertragen oder im Notfall Hilfe anfordern zu können.

In einer vorteilhaften Ausgestaltung ist vorgesehen, dass das mobile Handgerät eine Schnittstelle umfasst, welche zum Verbinden mit einer Ausleseeinheit ausgebildet ist. Eine derartige Ausleseeinheit kann beispielsweise mit einem Computersystem verbunden sein, welches der Patient oder der behandelnde Arzt oder Therapeut verwenden kann, um in dem Handgerät gespeicherte Daten auszulesen und zu analysieren. Die Schnittstelle könnte zudem für weitere Zwecke verwendbar ausgebildet sein, beispielsweise zur Verbindung mit einem Ladegerät zum Aufladen einer internen Batterie des Handgerätes. Die Schnittstelle kann für eine elektrische, optische, Funk- oder eine andere geeignete Verbindung zur Ausleseeinheit ausgestaltet sein.

Gemäß einer bevorzugten Weiterbildung ist eine Messwertauswertungseinheit vorgesehen, welche ausgebildet ist, eine Benutzeroberfläche zur Messwertauswertung bereitzustellen, die eingerichtet ist, eine Auswertung der gemessenen Blutzuckermesswerte zu unterstützen, wobei aus mehreren Blutzuckermesswerten ein Blutzuckermesswertverlauf ermittelt wird und ein oder mehrere Ausreißerwerte aus den Blutzuckermesswerten erkannt und mit den zugehörigen Bildern verknüpft werden. Bei solchen Ausreißern kann es sich sowohl um gegenüber einem Idealwert oder Mittelwert stark erhöhte als auch um stark verminderte Blutzuckermesswerte handeln.

Um sowohl Hyperglykämie als auch Hypoglykämie zu vermeiden, ist es Ziel einer modernen Diabetestherapie, den Blutzuckergehalt über einen möglichst langen Zeitraum konstant zu halten und allzu starke beziehungsweise plötzliche Abweichungen auszuschließen. Somit sind eine genaue Analyse der Ausreißerwerte und die Ermittlung ihrer Ursachen von großem Nutzen. Diese Analyse kann mit Hilfe der Benutzeroberfläche vorgenommen werden, welche beispielsweise auf einem Rechner des Therapeuten abläuft. Die Benutzeroberfläche kann zum Beispiel den zeitlichen Blutzuckermesswertverlauf anzeigen, so dass die Ausreißerwerte leicht sichtbar werden. Ferner kann sie es dem Benutzer ermöglichen, die erfassten Bilder nach bestimmten Parametern zu durchsuchen, insbesondere nach den Zeitpunkt der Bilderfassung, um sie mit den Ausreißerwerten zu korrelieren.

Vorteilhafterweise ist eine Messwertauswertungseinheit vorgesehen, welche ausgebildet ist, eine automatische Auswertung der gemessenen Blutzuckermesswerte derart durchzuführen, dass aus mehreren Blutzuckermesswerten ein Blutzuckermesswertverlauf ermittelt wird und ein oder mehrere Ausreißerwerte aus den Blutzuckermesswerten erkannt und mit den zugehörigen Bildern verknüpft werden. Die Verknüpfung kann in einer einfachen Ausführungsform dadurch geschehen, dass in einer Diagrammdarstellung der Blutzuckermesswerte, die den Ausreißerwerten zugehörigen oder zugeordneten Bilder oder deren Dateinamen angegeben werden. Alternativ oder zusätzlich kann zu diesem Zweck auch eine Verknüpfungstabelle erstellt werden.

Gemäß einer bevorzugten Ausgestaltung ist eine Bildauswertungseinheit vorgesehen, welche ausgebildet ist, eine Benutzeroberfläche zur Bildauswertung bereitzustellen, die eingerichtet ist, eine Auswertung der erfassten Bilder zu unterstützen, wobei Nährstoffeigenschaften von Speisen näherungsweise ermittelt werden, welche mittels der erfassten Bilder abgebildet sind. Bei den näherungsweise zu ermittelnden Nährstoffeigenschaften handelt es sich insbesondere um die Menge von relevanten Kohlehydraten, die in einer im Bild abgebildeten Nahrungszusammenstellung enthalten ist. Die Benutzeroberfläche kann beispielsweise Nährstofftabellen bereitstellen. Ferner können Bildbearbeitungsmodule bereitgestellt werden, um mit ihrer Hilfe die abgebildeten Nahrungsmittel besser sichtbar zu machen. Hier wie bei der Benutzeroberfläche zur Messwertauswertung können Expertensysteme zur Unterstützung eingesetzt werden.

Bevorzugterweise ist eine Bildauswertungseinheit vorgesehen, welche ausgebildet ist, eine automatische Auswertung der erfassten Bilder derart durchzuführen, dass Nährstoffeigenschaften von Speisen näherungsweise ermittelt werden, welche mittels der erfassten Bilder abgebildet sind. Dies kann mit Hilfe von Bilderkennungsprogrammen geschehen, beispielsweise nachdem eine automatische Bildbearbeitung beziehungsweise -aufbereitung vorgenommen wurde. Eine automatische Auswertung der gemessenen Blutzuckermesswerte wie auch eine automatische Auswertung der erfassten Bilder kann mittels neuronaler Netze oder anderer lernfähiger Systemen erfolgen.

Die Messwertauswertungseinheit und / oder die Bildauswertungseinheit können vollständig als Software oder zumindest teilweise als Hardware entweder als von der Blutzuckermesseneinheit und / oder der Bildaufnahmeeinheit getrennte Komponenten oder zumindest teilweise in dem gemeinsamen Gehäuse integriert verwirklicht sein.

Die Erfindung wird im Folgenden anhand von Ausführungsbeispielen unter Bezugnahme auf die Figuren erläutert. Hierbei zeigen:
- Fig. 1: eine Überwachungsvorrichtung in Form eines mobilen Handgerätes;
- Fig. 2: eine Rückansicht des mobilen Handgerätes aus der Fig. 1;
- Fig. 3: eine Rückansicht eines modularen Handgerätes;
- Fig. 4: eine Überwachungsvorrichtung mit einer Auswertungseinheit, einem mobilen Handgerät und einer Ausleseeinheit; und
- Fig. 5: ein Flussdiagramm eines Überwachungsverfahrens.

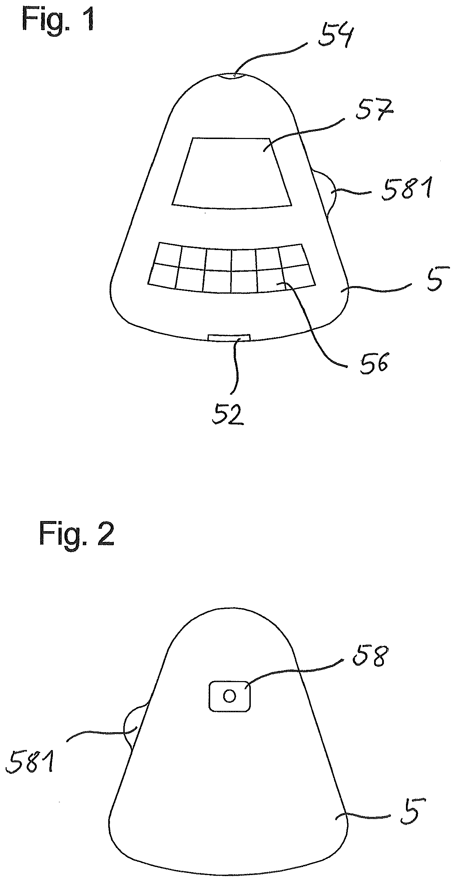

Die Fig. 1 zeigt ein mobiles Handgerät 5 in Draufsicht. Das mobile Handgerät 5 dient als Gehäuse für eine Überwachungsvorrichtung mit einer Blutzuckermesseinheit und einer Bildaufnahmeeinheit. Über einen Teststreifeneinschub 54 kann der Blutzuckermesseinheit ein Teststreifen mit einer Blutprobe zugeführt werden, deren Blutzuckergehalt zu bestimmen ist.

Das mobile Handgerät 5 umfasst ferner eine Bildanzeigeeinheit 57 und eine Eingabeeinheit 56, beispielsweise ein Tastaturfeld. Mit Hilfe dieser beiden Einheiten 56, 57 lässt sich die Überwachungsvorrichtung bedienen, beispielsweise indem mittels der Bildanzeigeeinheit 57 Menüpunkte dargestellt werden, welche durch einen Benutzer mittels der Eingabeeinheit 56 auswählbar sind, oder indem Befehle über die Eingabeeinheit 56 eingegeben und mittels der Bildanzeigeeinheit 57 dargestellt werden. Alternativ zu der in Fig. 1 gezeigten Bauweise des Handgerätes können die Bildanzeigeeinheit 57 und eine Eingabeeinheit 56 in einem Touchscreen vereint sein. Schließlich ist an dem mobilen Handgerät 5 in der Fig. 1 eine Schnittstelle 52 sichtbar, mit der eine Verbindung zu einer externen Ausleseeinheit hergestellt werden kann.

Die Bildanzeigeeinheit 57, beispielsweise eine LCD-Anzeige, kann zudem dazu dienen, mittels der Bildaufnahmeeinheit erfasste Bilder anzuzeigen. Wie in der Fig. 2 dargestellt, kann die Bildaufnahmeeinheit 58, deren Linse hier sichtbar ist, auf der Rückseite des mobilen Handgerätes 5 angeordnet sein. Wie bei einer herkömmlichen Digitalkamera kann dann der Benutzer das gewünschte Motiv auf der Bildanzeigeeinheit 57 betrachten und anschließend mit einer seitlich an dem mobilen Handgerät 5 angeordneten Bildauslösetaste 581 fotografieren.

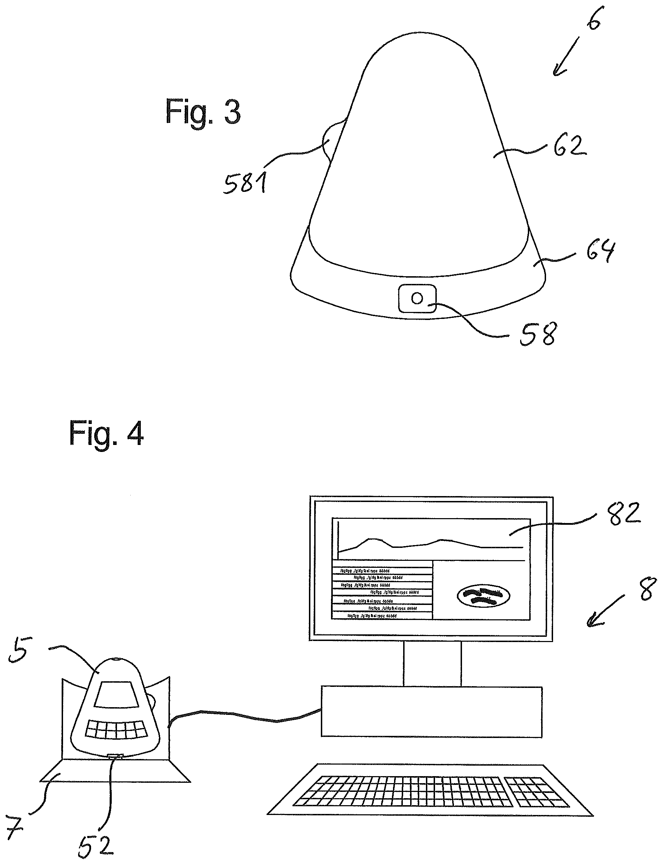

Eine Alternative zu der in der Fig. 2 dargestellten Ausführungsform für das mobile Handgerät 5, ist in der Fig. 3 dargestellt. Anstelle einer Integration der Blutzuckermesseinheit und der Bildaufnahmeeinheit 58 in einem gemeinsamen Gehäuse, ist in diesem Fall ein modulares Handgerät 6 vorgesehen. Hierbei befindet sich die Blutzuckermesseinheit in einem eigenen Blutzuckermessmodul 62, während die Bildaufnahmeeinheit 58 in einem separaten Bildaufnahmemodul 64 untergebracht ist. Die funktionelle Verbindung zwischen diesen beiden Modulen 62, 64 erfolgt dabei über eine in der Fig. 3 nicht sichtbare Schnittstelle.

Die Fig. 4 zeigt eine Anordnung mit einem Auswertesystem 8 zum Auswerten der mittels des mobilen Handgerätes 5 aufgenommenen Daten, also der gemessenen Blutzuckermesswerte und der erfassten Bilder. Hierzu ist mit dem Auswertesystem 8 eine Ausleseeinheit 7 verbunden, welche mit dem mobilen Handgerät 5 über seine Schnittstelle 52 gekoppelt werden kann. Während in der Fig. 4 eine Kabelverbindung zwischen der Ausleseeinheit 7 und dem Auswertesystem 8 dargestellt ist, können andere geeignete Verbindungsmittel zwischen dem mobilen Handgerät 5 und dem Auswertesystem 8 eingesetzt werden. Beispielsweise kann auch eine Verbindung über Funk und / oder via Internet erfolgen.

Das Auswertesystem 8, beispielsweise ein Computersystem, stellt eine Benutzeroberfläche 82 für die Unterstützung der Auswertung der gemessenen Blutzuckermesswerte und / oder der erfassten Bilder bereit. Alternativ oder zusätzlich hierzu kann das Auswertesystem die Auswertung der gemessenen Blutzuckermesswerte und / oder der erfassten Bilder teilweise oder vollständig automatisch durchführen. In der Fig. 4 ist das mobile Handgerät 5 lediglich eine Komponente der Überwachungsvorrichtung und das Auswertesystem 8 stellt eine getrennte Komponente dar.

Abgesehen davon, dass ein derartiges Auswertesystem 8 für eine Überwachungsvorrichtung nicht zwingend notwendig ist, können eine Messwertauswertungseinheit und / oder eine Bildauswertungseinheit in einem mobilen Handgerät 5 ähnlich dem aus den Fig. 1 und 2 oder in einem modularen Handgerät 6 ähnlich dem aus den Fig. 3 vorgesehen sein. Mit anderen Worten, eine oder beide Auswertungseinheiten können als Software oder Hardware verwirklicht in einem Handgerät 5, 6 mit integriert sein.

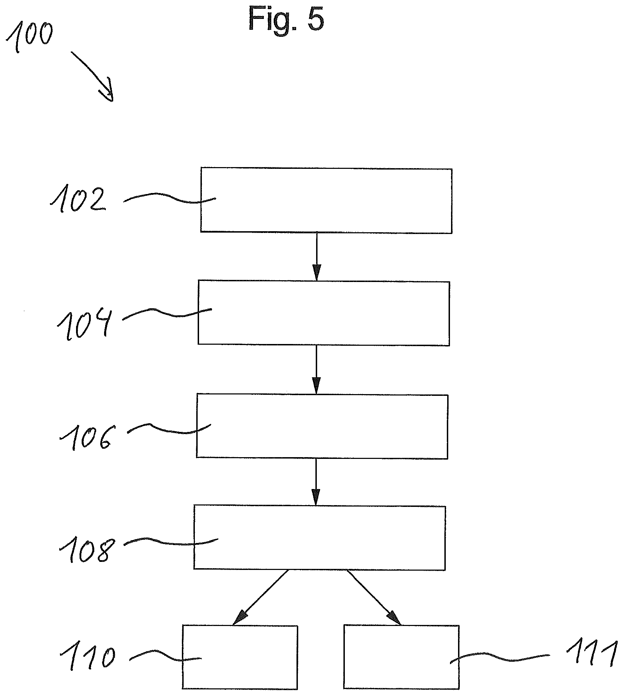

Schließlich zeigt die Fig. 5 ein Flussdiagramm für ein Überwachungsverfahren 100 für Blutzuckerwerte. Hierzu werden zunächst Blutzuckermesswerte gemessen 102. Anschließend oder der Messung vorausgehend werden ein oder mehrere Bilder erfasst 104, nämlich von Nahrungsmitteln oder Nahrungsmittel-Zusammenstellungen, deren Verzehr unmittelbar bevorsteht. Die aufgenommenen Bilder und die gemessenen Blutzuckermesswerte werden entweder in digitaler Form in eigenen Speicherelementen der Bildaufnahmeeinheit beziehungsweise der Blutzuckermesseinheit oder in einem gemeinsamen Speicher des mobilen Handgerätes 5 hinterlegt, oder sie werden unmittelbar an ein externes Auswertesystem 8 gesendet, welches dann neben der nachfolgend beschriebenen Auswertung 108 auch die Zuordnung 106 vornimmt.

Die erfassten Bilder werden anschließend den gemessenen Blutzuckermesswerten zugeordnet 106. Dies geschieht beispielsweise mit Hilfe von Zeitstempeln, mit denen die Bilder und die Blutzuckermesswerte versehen werden. Anschließend findet eine Auswertung 108 statt, die zum einen eine Auswertung der Blutzuckermesswerte 110 und zum anderen eine Auswertung der erfassten Bilder 111 umfasst.

Bei der Auswertung der Blutzuckermesswerten 110 geht es insbesondere darum, Ausreißerwerte zu ermitteln und zu bestimmen, welche der erfassten Bilder diesen Ausreißerwerten zugeordnet sind. Ziel es ist also, zu ermitteln, durch den Verzehr welcher Nahrungsmittel die Ausreißerwerte in den Blutzuckermesswerten verursacht sind. Demgegenüber geht es bei der ebenfalls optionalen Auswertung der erfassten Bilder darum, Nährstoffeigenschaften von auf den Bildern abgebildeten Nahrungsmitteln zumindest näherungsweise zu ermitteln, beispielsweise in Form von Broteinheiten.

Zwischen den vorangehend beschriebenen Verfahrensschritten des Überwachungsverfahrens 100 können auch zusätzliche Verfahrensschritte eingefügt sein, insbesondere hinsichtlich einer Übertragung von Blutzuckermesswerten oder Bildern in elektronischer Form zwischen dem mobilen Handgerät 5 und dem Auswertesystem 8.

### Bezugszeichenliste:

- 5: mobiles Handgerät
- 52: Schnittstelle
- 54: Teststreifeneinschub
- 56: Eingabeeinheit
- 57: Bildanzeigeeinheit
- 58: Bildaufnahmeeinheit
- 581: Bildauslösetaste

- 6: modulares Handgerät
- 62: Blutzuckermessmodul
- 64: Bildaufnahmemodul

- 7: Ausleseeinheit
- 8: Auswertesystem
- 82: Benutzeroberfläche

- 100: Überwachungsverfahren
- 102: Messen von Blutzuckermesswerten
- 104: Erfassen von Bildern
- 106: Zuordnen von Bildern zu Blutzuckermesswerten
- 108: Auswerten
- 110: Auswerten der Blutzuckermesswerte
- 111: Auswerten der Bilder

## Patentansprüche

1. Überwachungsvorrichtung für Blutzuckerwerte, umfassend eine Blutzuckermesseinheit (62) zum Messen von Blutzuckermesswerten,eine mit der Blutzuckermesseinheit (62) verbundene Bildaufnahmeeinheit (58, 64) zum Erfassen von Bildern und eine Zuordnungseinheit, welche ausgebildet ist zum Zuordnen der Bilder zu zugehörigen Blutzuckermesswerten.

2. Überwachungsvorrichtung nach Anspruch 1, **dadurch gekennzeichnet, dass** die Zuordnungseinheit ausgebildet ist zum Zuteilen von Zeitstempeln zu den Bildern und den Blutzuckermesswerten.

3. Überwachungsvorrichtung nach einem der vorangehenden Ansprüche, **gekennzeichnet durch** eine Bildanzeigeeinheit (57), welche ausgebildet ist zum Anzeigen der Bilder und / oder der Blutzuckermesswerte.

4. Überwachungsvorrichtung nach einem der vorangehenden Ansprüche, **dadurch gekennzeichnet, dass** die Blutzuckermesseinheit (62), die Bildaufnahmeeinheit(58, 64), die Zuordnungseinheit und / oder die Bildanzeigeeinheit in einem gemeinsamen Gehäuse untergebracht sind.

5. Überwachungsvorrichtung nach Anspruch 4, **dadurch gekennzeichnet, dass** das Gehäuse als mobiles Handgerät (5) ausgebildet ist.

6. Überwachungsvorrichtung nach Anspruch 5, **dadurch gekennzeichnet, dass** das mobile Handgerät eine Schnittstelle (52) umfasst, welche zum Verbinden mit einer Ausleseeinheit (7) ausgebildet ist.

7. Überwachungsvorrichtung nach einem der vorangehenden Ansprüche, **gekennzeichnet durch** eine Messwertauswertungseinheit (8), welche ausgebildet ist, eine Benutzeroberfläche (82) zur Messwertauswertung bereitzustellen, die eingerichtet ist, eine Auswertung der gemessenen Blutzuckermesswerte zu unterstützen, wobei aus mehreren Blutzuckermesswerten ein Blutzuckermesswertverlauf ermittelt wird und ein oder mehrere Ausreißerwerte aus den Blutzuckermesswerten erkannt und mit den zugehörigen Bildern verknüpft werden.

8. Überwachungsvorrichtung nach einem der Ansprüche 1 bis 6, **gekennzeichnet durch** eine Messwertauswertungseinheit (8), welche ausgebildet ist, eine automatische Auswertung der gemessenen Blutzuckermesswerte derart durchzuführen, dass aus mehreren Blutzuckermesswerten ein Blutzuckermesswertverlauf ermittelt wird und ein oder mehrere Ausreißerwerte aus den Blutzuckermesswerten erkannt und mit den zugehörigen Bildern verknüpft werden.

9. Überwachungsvorrichtung nach einem der vorangehenden Ansprüche, **gekennzeichnet durch** eine Bildauswertungseinheit (8), welche ausgebildet ist, eine Benutzeroberfläche (82) zur Bildauswertung bereitzustellen, die eingerichtet ist, eine Auswertung der erfassten Bilder zu unterstützen, wobei Nährstoffeigenschaften von Speisen näherungsweise ermittelt werden, welche mittels der erfassten Bilder abgebildet sind.

10. Überwachungsvorrichtung nach einem der Ansprüche 1 bis 8, **gekennzeichnet durch** eine Bildauswertungseinheit (8), welche ausgebildet ist, eine automatische Auswertung der erfassten Bilder derart durchzuführen, dass Nährstoffeigenschaften von Speisen näherungsweise ermittelt werden, welche mittels der erfassten Bilder abgebildet sind.

11. Überwachungsverfahren für Blutzuckerwerte, umfassend die folgenden Verfahrensschritte:
- Messen (102) von Blutzuckermesswerten;
- Erfassen (104) von Bildern; und
- Zuordnen (106) der Bilder zu zugehörigen Blutzuckermesswerten.

12. Überwachungsverfahren nach Anspruch 11, **dadurch gekennzeichnet, dass** die gemessenen Blutzuckermesswerte ausgewertet (108) werden, indem aus mehreren Blutzuckermesswerten ein Blutzuckermesswertverlauf ermittelt wird und ein oder mehrere Ausreißerwerte aus den Blutzuckermesswerten erkannt und mit den zugehörigen Bildern verknüpft werden (110).

13. Überwachungsverfahren nach Anspruch 11 oder 12, **dadurch gekennzeichnet, dass** eines oder mehrere der erfassten Bilder ausgewertet (108) werden, indem Nährstoffeigenschaften von Speisen näherungsweise ermittelt werden (111), welche mittels der erfassten Bilder abgebildet sind.

14. Überwachungsverfahren nach Anspruch 12 oder 13, **dadurch gekennzeichnet, dass** die Auswertung der gemessenen Blutzuckermesswerte und / oder die Auswertung der erfassten Bilder automatisch erfolgt.

## Claims

1. A monitoring device for blood glucose values, comprising a blood glucose measuring unit (62) for measuring blood glucose measured values and an image acquisition unit (58, 64) for acquiring images, which is connected to the blood glucose measuring unit (62), and an allocation unit which is designed for assigning the images to associated blood glucose measured values.

2. The monitoring device according to claim 1, **characterized in that** the allocation unit is designed for allocating time stamps to the images and the blood glucose measured values.

3. The monitoring device according to one of the preceding claims, **characterized by** an image display unit (57) which is designed to display the images and / or the blood glucose measured values.

4. The monitoring device according to one of the preceding claims, **characterized in that** the blood glucose measuring unit (62), the image acquisition unit (58, 64), the allocation unit and / or the image display unit are accommodated in a common housing.

5. The monitoring device according to claim 4, **characterized in that** the housing is designed as a mobile hand-held device (5).

6. The monitoring device according to claim 5, **characterized in that** the mobile hand-held device comprises an interface (52) which is designed for connection to a read-out unit (7).

7. The monitoring device according to one of the preceding claims, **characterized by** a measured value evaluation unit (8) which is designed to provide a user interface (82) for measured value evaluation, which is adapted to support an evaluation of the measured blood glucose measured values, wherein a blood glucose measured value progression is determined by several blood glucose measured values and one or several outlier values are detected among the blood glucose measured values and linked to the associated images.

8. The monitoring device according to one of claims 1 to 6, **characterized by** a measured value evaluation unit (8) which is designed to perform an automatic evaluation of the measured blood glucose measured values in such a way that a blood glucose measured value progression is determined by several blood glucose measured values and one or more outlier values are detected among the blood glucose measured values and linked to the associated images.

9. The monitoring device according to one of the preceding claims, **characterized by** an image evaluation unit (8) which is designed to provide a user interface (82) for image evaluation, which is adapted to support an evaluation of the acquired images, wherein nutrient properties of foods imaged by means of the acquired images are determined in approximation.

10. The monitoring device according to one of claims 1 to 8, **characterized by** an image evaluation unit (8) which is designed to perform an automatic evaluation of the acquired images in such a way that nutrient properties of foods imaged by means of the acquired images are determined in approximation.

11. A monitoring method for blood glucose values, comprising the following method steps:
- measuring (102) blood glucose measured values;
- acquiring (104) images; and
- assigning (106) the images to associated blood glucose measured values.

12. The monitoring method according to claim 11, **characterized in that** the measured blood glucose measured values are evaluated (108) by determining a blood glucose measured value progression by several blood glucose measured values and detecting one or more outlier values among the blood glucose measured values and linking them to the associated images (110).

13. The monitoring method according to claim 11 or 12, **characterized in that** one or more of the acquired images are evaluated (108) by determining approximately (111) nutrient properties of foods which are imaged by means of the acquired images.

14. The monitoring method according to claim 12 or 13, **characterized in that** the evaluation of the measured blood glucose measured values and / or the evaluation of the acquired images is performed automatically.

## Revendications

1. Un dispositif de surveillance pour des taux de glycémie, comprenant une unité (62) de mesure de la glycémie pour mesurer des valeurs mesurées de glycémie et une unité (58, 64) de capture d'image pour capturer des images qui est reliée à l'unité (62) de mesure de la glycémie et une unité d'allocation qui est conçue pour affecter les images à des valeurs mesurées de glycémie associées.

2. Le dispositif de surveillance selon la revendication 1, **caractérisé en ce que** l'unité d'allocation est conçue pour attribuer des estampilles temporelles aux images et aux valeurs mesurées de glycémie.

3. Le dispositif de surveillance selon l'une quelconque des revendications précédentes, **caractérisé par** une unité (57) d'affichage d'image qui est conçue pour afficher les images et / ou les valeurs mesurées de glycémie.

4. Le dispositif de surveillance selon l'une quelconque des revendications précédentes, **caractérisé en ce que** l'unité (62) de mesure de la glycémie, l'unité (58, 64) de capture d'image, l'unité d'allocation et / ou l'unité d'affichage d'image sont logées dans un boîtier commun.

5. Le dispositif de surveillance selon la revendication 4, **caractérisé en ce que** le boîtier est conçu comme un appareil (5) portatif mobile.

6. Le dispositif de surveillance selon la revendication 5, **caractérisé en ce que** l'appareil portatif mobile comprend une interface (52) qui est conçue pour le raccordement à une unité (7) de lecture.

7. Le dispositif de surveillance selon l'une quelconque des revendications précédentes, **caractérisé par** une unité (8) d'évaluation de valeur mesurée qui est conçue pour fournir une interface utilisateur (82) pour une évaluation de valeur mesurée, laquelle est adaptée pour soutenir une évaluation des valeurs mesurées de glycémie mesurées, dans lequel un cours de valeurs mesurées de glycémie est déterminé à partir de plusieurs valeurs mesurées de glycémie et une ou plusieurs valeurs aberrantes parmi les valeurs mesurées de glycémie sont détectées et liées aux images associées.

8. Le dispositif de surveillance selon l'une quelconque des revendications 1 à 6, **caractérisé par** une unité (8) d'évaluation de valeur mesurée qui est conçue pour effectuer une évaluation automatique des valeurs mesurées de glycémie mesurées de telle sorte qu'un cours de valeurs mesurées de glycémie est déterminé à partir de plusieurs valeurs mesurées de glycémie et une ou plusieurs valeurs aberrantes parmi les valeurs mesurées de glycémie sont détectées et liées aux images associées.

9. Le dispositif de surveillance selon l'une quelconque des revendications précédentes, **caractérisé par** une unité (8) d'évaluation d'image qui est conçue pour fournir une interface utilisateur (82) pour l'évaluation d'image, laquelle est adaptée pour supporter une évaluation des images capturées, dans lequel des propriétés nutritionnelles des aliments imagés au moyen des images capturées sont déterminées approximativement.

10. Le dispositif de surveillance selon l'une quelconque des revendications 1 à 8, **caractérisé par** une unité (8) d'évaluation d'image qui est conçue pour effectuer une évaluation automatique des images capturées de telle sorte que des propriétés nutritionnelles des aliments imagés au moyen des images capturées sont déterminées approximativement.

11. Un procédé de surveillance des taux de glycémie, comprenant les étapes de procédé suivantes:
- mesurer (102) de valeurs mesurées de glycémie;
- capturer (104) des images; et
- attribuer (106) des images à des valeurs mesurées de glycémie associées.

12. Le procédé de surveillance selon la revendication 11, **caractérisé en ce que** les valeurs mesurées de glycémie mesurées sont évaluées (108) en déterminant un cours de valeurs mesurées de glycémie à partir de plusieurs valeurs mesurées de glycémie et détectant une ou plusieurs valeurs aberrantes parmi les valeurs mesurées de glycémie et liant une ou plusieurs valeurs aberrantes aux images associées (110).

13. Le procédé de surveillance selon la revendication 11 ou 12, **caractérisé en ce qu'**une ou plusieurs des images capturées sont évaluées (108) en déterminant approximativement des propriétés nutritionnelles des aliments imagés au moyen des images capturées (111).

14. Le procédé de surveillance selon la revendication 12 ou 13, **caractérisé en ce que** l'évaluation des valeurs mesurées de glycémie mesurées et / ou l'évaluation des images capturées se fait automatiquement.
